# EUROPEAN PATENT SPECIFICATION

(11) **EP 1 696 840 B1**
(45) Date of publication and mention of the grant of the patent: **03.02.2010**
(21) Application number: 04801400.5
(22) Date of filing: 10.12.2004
(51) Int. Cl.: A61F 5/01

(54) **A SUPPORT FOR A LOWER LIMB PROVIDED WITH AN ORTHOPAEDIC SAFETY DEVICE**
STÜTZE FÜR EINE UNTERE GLIEDMASSE MIT EINER ORTHOPÄDISCHEN SICHERHEITSVORRICHTUNG
SUPPORT POUR MEMBRE INFERIEUR POURVU D'UN DISPOSITIF DE SECURITE ORTHOPEDIQUE

(30) Priority: 12.12.2003 IT BO20030746
(43) Date of publication of application: 06.09.2006
(73) Proprietor: Alma Mater Studiorum -Universita' di Bologna, 40126 Bologna (IT)
(72) Inventor: LORENZINI, Enrico, I-40141 Bologna (IT)
(74) Representative: Jorio, Paolo
(86) International application number: PCT/IB2004/004168
(87) International publication number: WO 2005/058211

(56) References cited:
- EP-A- 1 138 351
- GB-A- 2 260 495
- US-A- 4 688 559
- US-A- 4 946 156
- US-A- 5 653 680
- US-A1- 2003 004 444
- US-B1- 6 471 664

## Description

### TECHNICAL FIELD

The present invention relates to a support for a lower limb provided with an orthopaedic safety device.

### BACKGROUND ART

It is known that supports for the lower limbs (also referred to as "knee supports"), used for deambulation of subjects with motor difficulties due to a momentary or else permanent invalidity, are mechanical devices, which present the drawback of not envisaging any safety means in the event of a sudden and undesirable bending, or yielding, from the upright position to the bending position.

The sudden collapse of the load-bearing structure of the support on a statistical basis leads to a high percentage of cases of fracture of the femur, above all in subjects having at least one lower limb already weakened and in any case prevented from reacting.

As is known, a support for a lower limb comprises an upper portion, basically corresponding to the thigh, and a lower portion, roughly corresponding, to the calf and to the ankle.

Between the upper portion and the lower portion, at the height of the knee, there is present a hinge that enables bending of the lower portion with respect to the upper one, as is peculiar to the human leg. During locomotion, the upper and lower portions of the support are rigidly aligned with respect to one another to enable the leg of the user to assume the upright position. For this purpose, the hinge of articulation between the two portions is immobilized by a brake that prevents the articulation of one portion with respect to the other.

In the case where the user has to sit down, he will just have to release the brake so as to enable articulation of the lower portion with respect to the upper one, precisely as occurs for a leg without supports.

However, there may occur an accidental yielding of the support if the brake is not perfectly engaged.

If the brake is disengaged suddenly for accidental reasons without there being provided any safety apparatus, the collapse is total and immediate in so far as it is the very weight of the person that bends the knee support violently.

In this context there occur numerous cases of femoral fractures or of other injuries of lesser importance, which even so remain serious, especially in subjects with lower limbs that are already debilitated.

In US-A-4 946 156 a support for a lower limb is described. Such a support comprises an upper portion, binding the thigh of a user, and a lower portion housing the ankle and the calf of the user. Furthermore, the supports comprises an articulation device between the upper portion and the lower portion. The articulation device is provided with braking means.

However, in the support described in US-A-4 946 156 isn't envisaged any orthopaedic safety device designed to react by braking the collapse of the upper portion onto the lower portion in the event of sudden and undesirable release of the braking means.

There has consequently emerged the need to envisage for supports of this type an orthopaedic safety device that will be able to decelerate the bending of the upper portion with respect to the lower portion in order to exert at the same time a braking action on the fall of the subject so that the injury will be annulled or in any case limited.

### DISCLOSURE OF INVENTION

The subject of the present invention is, therefore, to provide an orthopaedic safety device of simple conception and construction to be applicable to any kind of support for a lower limb.

Consequently, according to the present invention there is provided a support for a lower limb according to the characteristics contained in Claim 1.

### BRIEF DESCRIPTION OF THE DRAWINGS

The present invention will now be described with reference to the annexed plate of drawings, which illustrate a non-limiting example of embodiment thereof and in which:
- Figure 1 illustrates a front view of a support for a lower limb according to the invention;
- Figure 2 shows a rear view of the support of Figure 1;
- Figure 3 shows a side view of the support of Figures 1 and 2, the support being in its completely erect configuration;
- Figure 4 illustrates a side view of the support of Figures 1 and 2, the support being in its configuration partially bent at the height of the knee;
- Figure 5 shows a side view of some enlarged details of the support represented in Figures 1-4, a brake being engaged for immobilizing the rotation of one portion with respect to the other;
- Figure 6 shows a side view of the same details illustrated in Figure 5, the immobilizing brake being disengaged; and
- Figure 7 illustrates two enlarged views of an orthopaedic safety device used in the support forming the subject of the present invention.

### BEST MODE FOR CARRYING OUT THE INVENTION

In the attached figures, the reference number 10 designates a support for a lower limb in accordance with the present invention.

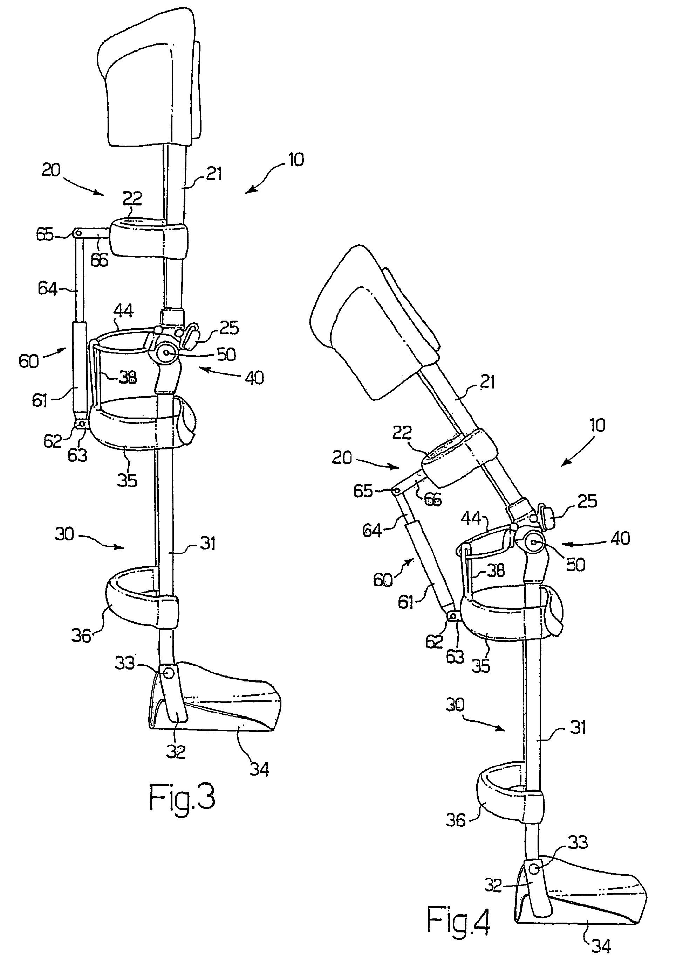

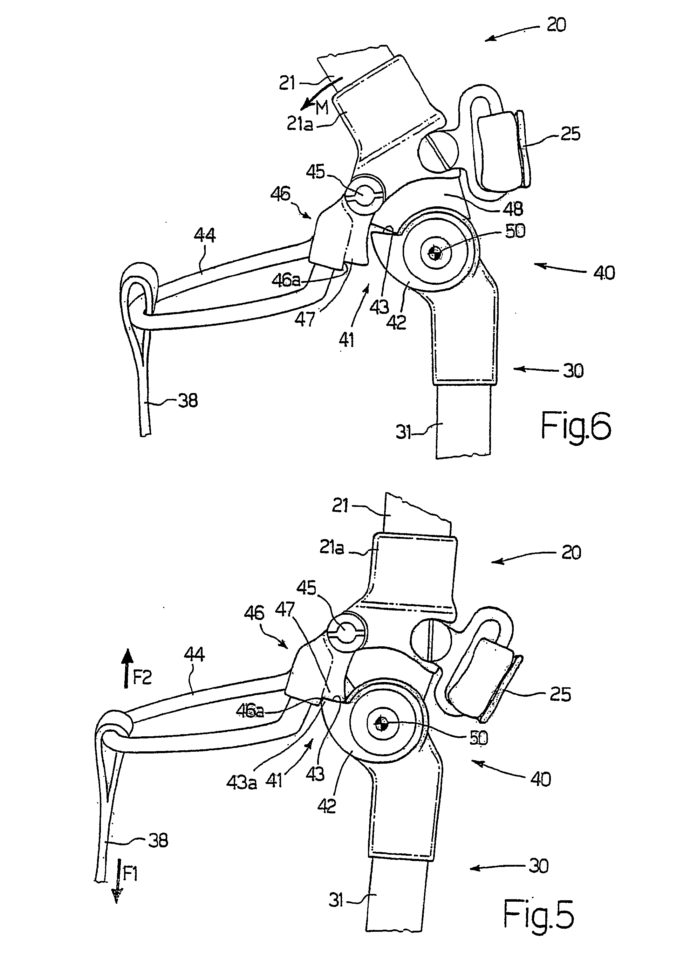

The support 10 comprises an upper portion 20 and a lower portion 30 hinged to one another by means of an articulation device 40, whereby bending of the lower portion 30 with respect to the upper portion 20 is enabled, as is shown, for example, in Figures 4 and 6 and as will emerge more clearly from what follows.

The upper portion 20 in turn comprises two metal rods 21, set substantially parallel to one another, joined transversely by a first thigh cuff 22 and a second thigh cuff 23, which functions as an ischial rest.

Welded to the lower end of each rod is a corresponding metal element 21a.

The two thigh cuffs 22 and 23 are also made of metal, are substantially semicircular in shape and are coated with a padding 22a and 23a, respectively, to increase the comfort of the user.

The two thigh cuffs 22, 23 consequently define, in a traditional way, a housing 23b, in which there is inserted the upper portion of the disabled leg (not shown) so that it can be held firmly erect during locomotion.

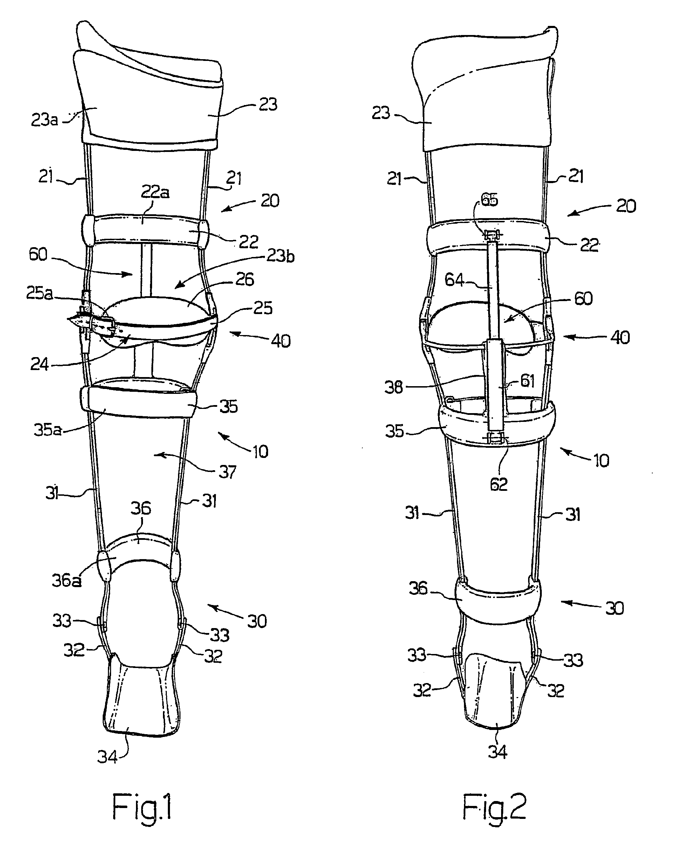

The front part of the housing 23 is closed by a closing and containment device 24 comprising a strap 25 provided with a corresponding buckle 25a (Figure 1). The strap 25 passes through two slots (not shown) made in a padded guard 26, by means of which the desired containment of the user's thigh is obtained.

In turn, the lower portion 30 comprises two metal rods 31 slightly convergent downwards. Hinged via a pin 33 to the rods 31, at their bottom ends, is a bracket 32. Furthermore, fixedly connected to the bracket 32 is a footrest 34, on which the user's foot (not shown) rests in use.

As was said for the upper portion 20, the two rods 31 of the lower portion 30 are joined transversely by a first calf cuff 35 and by a second calf cuff 36.

The two calf cuffs 35 and 36 are also made of metal, have a substantially semicircular shape, and are coated by a padding 35a and 36a, respectively, to increase the comfort of the user.

The two calf cuffs 35, 36 consequently define, in a traditional way, a housing 37, in which the lower portion of the disabled leg (not shown) is inserted.

As shown in particular in Figures 5 and 6, the articulation device 40 comprises an articulation pin 50, about which relative rotation of the lower portion 30 with respect to the upper portion 20 can take place.

Also belonging to the articulation device 40 is a brake 41, which in turn comprises a plate 42 fixed to the lower portion 30. The plate 42 has a substantially horizontal contrast element 43.

The brake 41 further comprises a bracket 44, hinged to each element 21a by means of a respective articulation pin 45. Fixed to the bracket 44 is a pawl-and-ratchet device 46, the pawl of which 47 rests on the contrast element 43 when the upper portion 20 is aligned to the lower portion 30, i.e., in the case where the user is walking or is in an upright position. Furthermore, the end 43a of the contrast element 43 is pushed against a shoulder 46a of the pawl-and-ratchet device 46.

The pawl 47 is gripped firmly on the contrast element 43 by the action of an elastic strap 38 extending between the calf cuff 35 and the bracket 44. The elastic strap 38 pulls the bracket 44 downwards with a force F1.

It should incidentally be said that, even though for convenience of exposition reference is made to just one pawl-and-ratchet device 46, there are evidently provided two pawl-and-ratchet devices 46, one for each rod 21.

To release the brake 41 voluntarily, the user just has to pull the bracket 44 upwards, producing a force F2 greater than the force F1 exerted by the elastic strap 38. On account of this action, the pawl 47 of the pawl-and-ratchet device 46 slides on the contrast element 43, disengaging the pawl-and-ratchet device 46 itself from the plate 42.

Once release has occurred, it is possible to bring about a relative rotation of the upper portion 20 with respect to the lower portion 30 in the direction indicated by the arrow M, as represented in Figures 4 and 6. As shown in Figure 6, once the brake 41 has been disengaged, the contrast element 43 is free to rotate also in the recess 48 made in the element 21a.

The support 10 described so far belongs to the known art.

Forming the subject of the present invention is an orthopaedic safety device 60 shown in greater detail in Figures 3, 4 and 7.

The device 60 comprises a bottom cylinder 61, hinged by means of a pin 62 to a cantilever 63 fixed to the calf cuff 35. The bottom cylinder 61 is filled with a liquid OL presenting a high degree of viscosity.

The device 60 further comprises a top cylinder 64 hinged, by means of a pin 65, to a cantilever 66 fixed to the thigh cuff 22.

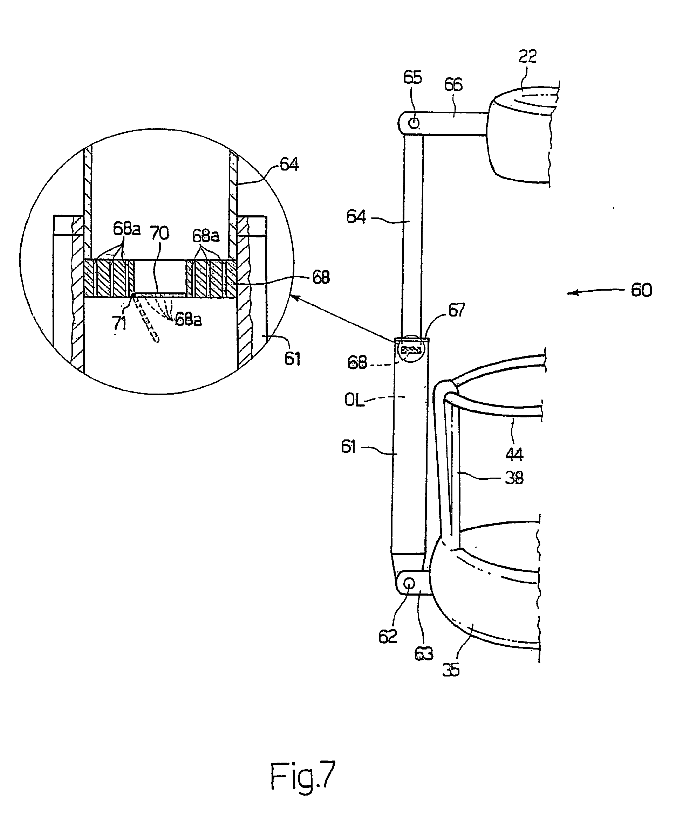

Furthermore, as shown in particular in Figure 7, the diameter of the cylinder 64 is smaller than that of the cylinder 61 so that the latter may house at least partially the cylinder 64 itself. Furthermore, the cylinder 61 is closed by a sealing disk 67, which prevents the viscous liquid OL from coming out and which enables sliding of the cylinder 64 within the cylinder 61.

One end of the cylinder 64 is advantageously provided with a filter 68, which has a plurality of holes 68a, through which the viscous liquid OL passes from the cylinder 61 to the cylinder 64 when the system passes from the configuration shown in Figure 3 to the configuration illustrated in Figure 4.

The passage of viscous liquid OL produces a braking effect on the movement of the upper portion 20 with respect to the lower portion 30. In fact, the oil flows at a reduced speed through the filter 68, which presents a particular mechanical resistance and envisages, as has been said, a plurality of holes 68a of a microscopic nature or in any case of a nature such that the introduction of the cylinder 64 into the cylinder 61 is decelerated.

In other words, the device 60 behaves as a true hydraulic damper of the angular displacements of the upper portion 20 with respect to the lower portion 30.

Furthermore, as is shown in the enlarged view of Figure 7, to enable a convenient reversibility of the mechanism when the support 10 is brought back into the erect position of Figure 3, the filter 68 is provided with a flap 70 hinged to the main body of the filter 68 by means of a hinge 71. As may be noted, the flap 70 is a one-way valve, which enables fast passage of the viscous liquid OL from the cylinder 64 to the cylinder 61 to bring the system back quickly into its initial conditions. It is evident that also the flap 70 can be provided with holes 68a (as shown in Figure 7) to enable also a limited passage of viscous liquid OL from the cylinder 61 to the cylinder 64 when there is a transition from the configuration represented in Figure 3 to the one illustrated in Figure 4, i.e. during the step of bending of the support 10. The flap 70 opens as a result of the weight precisely and exclusively when the cylinder 61 is empty, whilst the cylinder 64 is at least partially full of viscous liquid OL.

In an alternative embodiment (not represented), the entire filter 68 is hinged to the inside wall of the cylinder 64, and hence, in this case, it is the entire filter 68 that functions as a flap 70.

## Claims

1. A support (10) for a lower limb comprising an upper portion (20), binding the thigh of a user, and a lower portion (30), housing the ankle and the calf of the user;
- said support (10) comprising articulation means (40) between said upper portion (20) and said lower portion (30), said articulation means (40) being provided with braking means (41);
- said support (101) being **characterized in that**
it comprises an orthopaedic safety device (60) which decelerates the bending of said upper portion (20) onto said lower portion (30) in the event of sudden and undesirable release of said braking means (41) .

2. The support (10) according to Claim 1, in which said orthopaedic safety device (60) mechanically connects said upper portion (20) to said lower portion (30).

3. The support (10) according to Claim 2, in which said orthopaedic safety device (60) comprises a bottom cylinder (61), mechanically conducted to said lower portion (30), inside which a top cylinder (64) connected mechanically to said upper portion (20) slides in a fluid-tight way.

4. The supports (10) according to Claim 3, in which between said bottom cylinder (61) and said top cylinder (64) is present a braking liquid (OL).

5. The support (10) according to Claim 4, in which one end of said cylinder (64) is provided with a filter (68), through which, in the braking phase, the braking liquid (OL) passes from said cylinder (61) to said cylinder (64), the passage of braking liquid (OL) producing the desired braking effect on the movement of said upper portion (20) with respect to said lower portion (30).

6. The supports (10) according to any one of Claims 3-5, in which said bottom cylinder (61) is hinged, by means of a pin (62), to a cantilever (6B) fixed to a calf cuff (35) belonging to said lower portion (30).

7. The support (10) according to any one of Claims 3-6, in which said top cylinder (64) is hinged, by means of a pin (65), to a cantilever (66) fixed to a thigh cuff (22) belonging to said upper portion (20).

8. The support (10) according to Claim 5, in which at least one portion (70) of said filter (68) is hinged by means of a hinge (71), said at least one portion (70) functioning as a one-way valve to enable fast reversibility of the mechanism.

## Patentansprüche

1. Eine Stütze (10) für einen Unterschenkel, wobei die Stütze ein Oberteil (20), zum Anbinden an den Oberschenkel eines Nutzers, und ein Unterteil (30) zum Aufnehmen des Fußgelenks und der Wade des Nutzers, aufweist;
- wobei die Stütze (10) Gelenkmittel (40) aufweist zwischen dem Oberteil (20) und dem Unterteil (30), wobei die Gelenkmittel (40) mit Bremsmitteln (41) versehen sind;
- wobei die Stütze (10) **dadurch gekennzeichnet ist, dass** sie eine orthopädische Sicherheitseinrichtung (60) aufweist, die das Biegen des Oberteils (20) bezüglich des Unterteils (30) abbremst im Fall einer plötzlichen und unerwünschten Freigabe der Bremsmittel (41).

2. Stütze (10) nach Anspruch 1, in der die orthopädische Sicherheitseinrichtung (60) das Oberteil (20) mechanisch mit dem Unterteil (30) verbindet.

3. Stütze (10) nach Anspruch 2, wobei die orthopädische Sicherheitseinrichtung (60) einen unteren Zylinder (61) aufweist, der mit dem Unterteil (30) mechanisch verbunden ist, in dem ein oberer Zylinder (64), der mechanisch mit dem Oberteil (20) verbunden ist, in einer strömungsdichten Art und Weise gleitet.

4. Stütze (10) nach Anspruch 3, wobei zwischen dem unteren Zylinder (61) und dem oberen Zylinder (64) eine Bremsflüssigkeit (OL) vorgesehen ist.

5. Stütze (10) nach Anspruch 4, wobei ein Ende des Zylinders (64) mit einem Filter (68) versehen ist, durch den während der Bremsphase die Bremsflüssigkeit (OL) von dem Zylinder (61) zu dem Zylinder (64) hindurchgeht, wobei das Hindurchströmen der Bremsflüssigkeit (OL) den gewünschten Bremseffekt bezüglich der Bewegung des Oberteils (20) zu dem Unterteil (30) bewirkt.

6. Stütze (10) nach einem der Ansprüche 3 bis 5, wobei der untere Zylinder (61) mittels eines Stiftes (62) an einem Ausleger (68) angelenkt ist, der an einer Wadenmanschette (35) befestigt ist, die zu dem Unterteil (30) gehört.

7. Stütze (10) nach einem der Ansprüche 3 bis 6, wobei der obere Zylinder (64) durch einen Stift (65) an einem Ausleger (66) angelenkt ist, der an einer Oberschenkelmanschette (22) befestigt ist, die zu dem Oberteil (20) gehört.

8. Stütze (10) nach Anspruch 5, wobei wenigstens ein Teil (70) des Filters (68) mittels eines Gelenks (71) angelenkt ist, wobei der wenigstens eine Teil (70) als ein Einwegventil arbeitet, um eine schnelle Reversibilität des Mechanismus zu ermöglichen.

## Revendications

1. Support (10) pour membre inférieur comprenant une partie supérieure (20) reliant la cuisse d'un utilisateur et une partie inférieure (30), recevant la cheville et le mollet de l'utilisateur ;
- ledit support (10) comprenant des moyens d'articulation (40) entre ladite partie supérieure (20) et ladite partie inférieure (30), lesdits moyens d'articulation (40) étant dotés de moyens de freinage (41) ;
- ledit support (10) étant **caractérisé en ce qu'**il comprend un dispositif de sécurité orthopédique (60) qui ralentit la courbure de ladite partie supérieure (20) sur ladite partie inférieure (30) dans le cas d'un relâchement soudain et indésirable desdits moyens de freinage (41).

2. Support (10) selon la revendication 1, dans lequel ledit dispositif de sécurité orthopédique (60) relie mécaniquement ladite partie supérieure (20) à ladite partie inférieure (30).

3. Support (10) selon la revendication 2, dans lequel ledit dispositif de sécurité orthopédique (60) comprend un cylindre inférieur (61) relié mécaniquement à ladite partie inférieure (30) à l'intérieur duquel un cylindre supérieur (64) relié mécaniquement à ladite partie supérieure (20) glisse de façon étanche aux fluides.

4. Support (10) selon la revendication 3, dans lequel entre ledit cylindre inférieur (61) et ledit cylindre supérieur (64) se trouve un liquide de freinage (OL).

5. Support (10) selon la revendication 4, dans lequel une extrémité dudit cylindre (64) est dotée d'un filtre (68) par lequel, dans la phase de freinage, le liquide de freinage passe dudit cylindre (61) audit cylindre (64), le passage du liquide de freinage (OL) produisant l'effet de freinage souhaité sur le mouvement de ladite partie supérieure (20) par rapport à ladite partie inférieure (30).

6. Support (10) selon l'une quelconque des revendications 3 à 5, dans lequel ledit cylindre inférieur (61) est articulé au moyen d'une broche (62) à un élément en porte-à-faux (6B) fixé à une coiffe du mollet (35) faisant partie de ladite partie inférieure (30).

7. Support (10) selon l'une quelconque des revendications 3 à 6, dans lequel ledit cylindre supérieur (64) est articulé au moyen d'une broche (65) à un élément en porte-à-faux (66) fixé à une coiffe de cuisse (22) faisant partie de ladite partie supérieure (20).

8. Support (10) selon la revendication 5, dans lequel au moins une partie (70) dudit filtre (68) est articulée au moyen d'une charnière (71), au moins ladite partie (70) fonctionnant comme un clapet unidirectionnel pour permettre une réversibilité rapide du mécanisme.
